# EUROPEAN PATENT SPECIFICATION

(11) **EP 2 376 683 B1**
(45) Date of publication and mention of the grant of the patent: **11.06.2014**
(21) Application number: 10700773.4
(22) Date of filing: 08.01.2010
(51) Int. Cl.: D01F 8/14, A61L 9/12

(54) **HYDROPHILIC POROUS WICKS FOR VAPORIZABLE MATERIALS**
HYDROPHILE PORÖSE DOCHTE FÜR VERDAMPFBARE MATERIALIEN
MÈCHES POREUSES HYDROPHILES POUR SUBSTANCES VAPORISABLES

(30) Priority: 09.01.2009 US 143646 P; 18.05.2009 US 179141 P
(43) Date of publication of application: 19.10.2011
(73) Proprietor: Porex Corporation, Fairburn, GA 30213 (US)
(72) Inventor: ARTHUR, Michael, Douglasville GA 30213 (US); SPORRER, Kevin, Newnan GA 30265 (US); MAO, Guoqiang, Peachtree City GA 30269 (US)
(74) Representative: Dey, Michael
(86) International application number: PCT/US2010/020514
(87) International publication number: WO 2010/081013

(56) References cited:
- WO-A2-03/080904
- WO-A2-2007/149966
- US-A- 5 620 641
- US-A1- 2005 189 292
- US-A1- 2008 056 691

## Description

### FIELD OF THE INVENTION

The present invention is in the field of hydrophilic porous wicks, biodegradable porous wicks and biodegradable porous media.

### BACKGROUND

A fragrance device that uses multiple reed wicks to release fragrance is known. The device generally contains a jar partially filled with a fragrance liquid and a number of reed wicks. In these devices, the fragrance is typically dissolved in organic solvents, which are used as carriers. A number of reed wicks are placed in the jar with a portion of the length of the reed wicks submerged in the fragrance solution. The fragrance solution wicks into the reed wicks and rises by capillary forces through the length of the reed wicks. Fragrance is then released into the air by fragrance evaporation from the reed wick surface. This type of device is energy efficient because it uses no heating element, motorized fans, or atomizing pumps. For environmental compatibility, there is a trend to use water based fragrances instead of solvent based fragrances and to use biodegradable wicks instead of non-biodegradable based wicks. Traditional reed wicks are made of bamboo or rattan, which are biodegradable. However, bamboo or rattan based reeds lack uniformity and their wicking properties vary depending on the sources of the bamboo or rattan. Aqueous based fragrance fluids do not function well with traditional reed wicks. The wick speed and delivery rates of aqueous based fragrance fluids are not satisfactory in real application for traditional reed wicks. Synthetic polymer wicks have been used for fragrance wicks, such as polyester and polyolefin material based fiber wicks. However, these synthetic fabric materials are not biodegradable and may have a long term negative environmental impact once they are disposed. There is a need for a biodegradable, hydrophilic wick that could wick aqueous based fragrance liquids faster, higher and more uniformly to deliver more fragrance into the environment than the current traditional reed wicks. Consumers prefer colored wicks in many occasions for decorative purposes, such as a natural wood color for the wicks. Colors used for wicks need to be stable and should not fade during the application. There is a need for a biodegradable, hydrophilic colored wick that could wick aqueous based fragrance liquids faster, higher and more uniformly to deliver more fragrance into the environment than the current traditional reed wicks.

In particular, the present invention relates to a hydrophilic porous wick comprising sintered synthetic bicomponent fibers having pores between the sintered synthetic bicomponent fibers, wherein the synthetic bicomponent fibers comprise PLA/PLA bicomponent fibers.

### SUMMARY OF THE INVENTION

This invention provides a hydrophilic fiber wick. This invention also provides a biodegradable fiber wick. This invention also provides a hydrophilic, biodegradable fiber wick. This invention provides biodegradable porous media. The wicks of the present invention may be made from different materials and may be used for delivering a vaporizable material into an environment, such as a room environment. The wicks of the present invention may be made from different materials and may be used for delivering fragrance into an environment, such as a room environment. Aqueous based fragrances in this invention are fragrance formulations that have a water composition over 50% by weight. The wicks of the present invention may also deliver non-aqueous based fragrances into an environment. The wicks of the present invention may also deliver other vaporizable materials into an environment. These wicks may be made of synthetic fiber materials, or a blend of synthetic fiber materials and natural fiber materials. In one embodiment, synthetic fiber materials comprise synthetic bicomponent fibers. In another embodiment, synthetic fiber materials comprise synthetic bicomponent fibers and natural monocomponent fibers or synthetic monocomponent fibers. Synthetic fibers may be biodegradable or non-biodegradable. In another embodiment, synthetic bicomponent fibers are combined with natural fibers to form a wick. In one embodiment, natural fiber materials comprise cotton. In one embodiment, either the synthetic fiber materials, the natural fiber materials or both may be colored. The wicks of the present invention may be colored.

This invention provides a synthetic biodegradable fiber fragrance wick for delivering fragrance into an environment, such as a room environment.

This invention also provides a composite biodegradable fiber fragrance wick comprising a synthetic biodegradable fiber material and a natural biodegradable fiber material for delivering fragrance into an environment, such as a room environment.

This invention also provides a method of delivering a vaporizable material, such as a fragrance, into the atmosphere comprising placing a hydrophilic porous wick comprising sintered synthetic bicomponent fibers having pores between the sintered synthetic bicomponent fibers into a fluid containing a vaporizable material and permitting the fluid containing the vaporizable material to wick through the hydrophilic porous wick and release into the atmosphere. This invention also provides a method of delivering a vaporizable material, such as a fragrance, into the atmosphere comprising placing a hydrophilic porous wick comprising sintered synthetic bicomponent fibers having pores between the sintered synthetic bicomponent fibers, and natural monocomponent fibers or synthetic monocomponent fibers, into a fluid containing a vaporizable material and permitting the fluid containing the vaporizable material to wick through the hydrophilic porous wick and release into the atmosphere.

This invention also provides a composite biodegradable fiber fragrance wick comprised of a majority of synthetic biodegradable fiber materials and a minority of natural biodegradable fiber materials for delivering fragrance into an environment, such as a room environment.

In one embodiment, the wicks are made from a synthetic sinterable bicomponent fiber. In another embodiment, the wicks are made from a two component fiber blend of a synthetic sinterable bicomponent fiber and a monocomponent fiber that is carded into a sliver which is subsequently subjected to heat and pressure in an oven pultrusion process. A die on the output side of the oven forms rods of desired diameter that are subsequently cut into wicks. The sheath or core of the bicomponent fiber and/or the monocomponent fiber may be colored. The majority of the fiber blend is composed of the bicomponent synthetic fiber (over 50 wt% to about 95 wt%). The bicomponent synthetic fiber is at least more than 50 wt%, 60 wt%, 70 wt%, 80 wt%, 90 wt% or 95 wt% of the weight of the wick. The minor component of the fiber blend is a monocomponent fiber which may be colored. This monocomponent fiber can be a synthetic fiber or natural fiber. The monocomponent fiber can be a solution dyed synthetic, reactive dyed synthetic, reactive dyed cotton, genetically modified colored cotton or naturally colored cotton. In another embodiment, wicks are made using solution dyed bicomponent fiber. In one embodiment, for carding purposes, the bicomponent fibers and monocomponent fibers should be of similar denier (for example about 2-12 denier), length (about 12.7 mm (0.5 inches) to about 76.2 mm (3.0 inches)), and crimp. The colored monocomponent fibers have a melt or decomposition temperature at least about 10°C higher than that of the bicomponent sheath material.

The sliver is bonded together by using an oven pultrusion process. The oven thermally bonds (melts) the sheath material of the bicomponent fibers to other bicomponent fibers and to the non binding monocomponent fibers. This process produces a cylindrical sintered porous matrix. A die compresses and shapes this matrix into rods that are subsequently air cooled and cut to length.

To improve the wicking of vaporizable materials, such as water based fragrances, procedures have been developed to increase the surface energy of a fiber wick material. One way to increase this surface energy is to treat wicks using plasma. This can be a batch process at low pressure or an inline process at or above atmospheric pressure. A number of gases can be energized to react with the surface of the fiber to create hydrophilic moieties. Gases include, but are not limited to, oxygen, air, nitrogen, argon and a combination thereof. Various exposure times, pressures, and energies are used during the plasma process depending on the desired product requirements. Experimental results have shown that plasma treating sintered fiber wicks increases the capillary rise height and rate of rise of water based fragrance fluids within the fiber wick. In several embodiments, deionized water moved against gravity at the rate of over 10.16 mm/minute (0.4 inches/minute) in the initial 50.8 mm (2 inches) of the wick. The poly (lactic acid) (PLA)/PLA bicomponent fiber is a type of polyester, and polyester materials respond well to oxygen plasma treatment processes both in terms of hydrophilic wicking improvement and shelf life.

In one embodiment, the wick is comprised of synthetic sinterable hydrophilic bicomponent fibers. In a specific embodiment, all fiber compositions in the wicks are hydrophilic. In another specific embodiment, some, most, or all of the synthetic sinterable hydrophilic bicomponent fibers are colored.

In one embodiment, the wick is comprised of synthetic sinterable biodegradable bicomponent fibers. In a specific embodiment, all fiber compositions in the wicks are biodegradable. In another specific embodiment, some, most or all of the synthetic sinterable biodegradable bicomponent fibers are colored.

In one embodiment, the wick is comprised of synthetic sinterable hydrophilic bicomponent fibers and a colored naturally biodegradable fiber. In a specific embodiment, the biodegradable fiber is dyed or naturally colored.

In one embodiment, the wick is comprised of synthetic sinterable hydrophilic bicomponent fibers and a colored naturally biodegradable cotton fiber. In a specific embodiment, the colored cotton is dyed or naturally colored.

In one embodiment, the wick is comprised of synthetic sinterable biodegradable bicomponent fibers and a colored naturally biodegradable fiber. In a specific embodiment, the colored naturally biodegradable fiber is dyed or naturally colored.

In one embodiment, the wick is comprised of synthetic sinterable biodegradable bicomponent fibers and a colored naturally biodegradable cotton fiber. In a specific embodiment, the colored cotton is dyed or naturally colored.

In yet another embodiment, the wick is comprised of synthetic sinterable biodegradable bicomponent fibers and non-biodegradable bicomponent fibers, wherein the biodegradable biocomponent fiber is the majority component in the wick.

In yet a further embodiment, the wick is comprised of synthetic sinterable biodegradable bicomponent fibers and colored hydrophilic cellulosic fibers.

In another embodiment, the wick is comprised of synthetic sinterable biodegradable bicomponent fibers and colored hydrophilic synthetic fibers. The colored hydrophilic synthetic fibers may be biodegradable or not biodegradable.

The wicks of the present invention may be optionally activated by plasma treatment for improved hydrophilicity. The wicks of the present invention may be optionally activated by employing finishing agents for improved hydrophilicity. In one embodiment, finishing agents may be applied to the bicomponent fibers. In another embodiment, finishing agents are present in the commercially available bicomponent fibers. The wicks of the present invention can wick vaporizable materials, such as aqueous or non-aqueous-based fragrances, and evaporate the fragrances into the air without the use of heat, convection air, or atomization.

The biodegradable wick composition can be also used in the applications listed in PCT/US02/11829 (WO 03/080904), which include applications as filtration media for pipette tip filter, as an in line filter, as writing instrument nibs, seal sealing filtration media and in other disposable filtration and barrier applications.

### BRIEF DESCRIPTION OF THE FIGURES

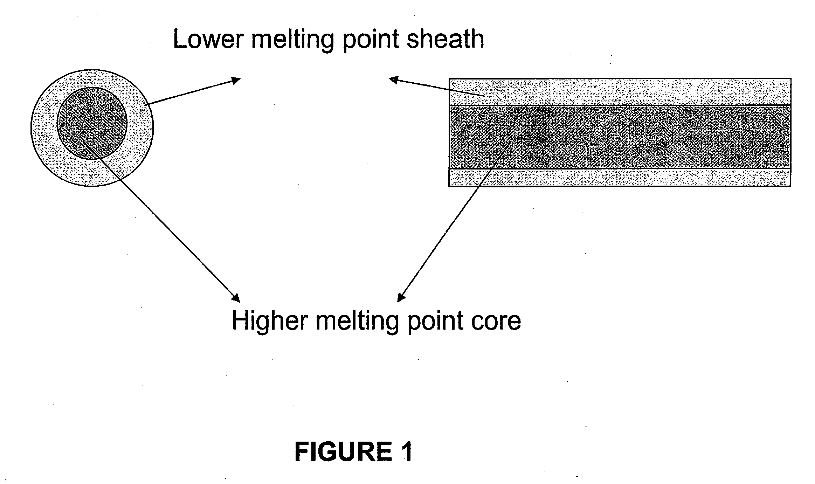

Figure 1 is a schematic representation of longitudinal view and cross-sectional view of one embodiment of a staple bicomponent fiber with a lower melting point sheath and a higher melting point core.

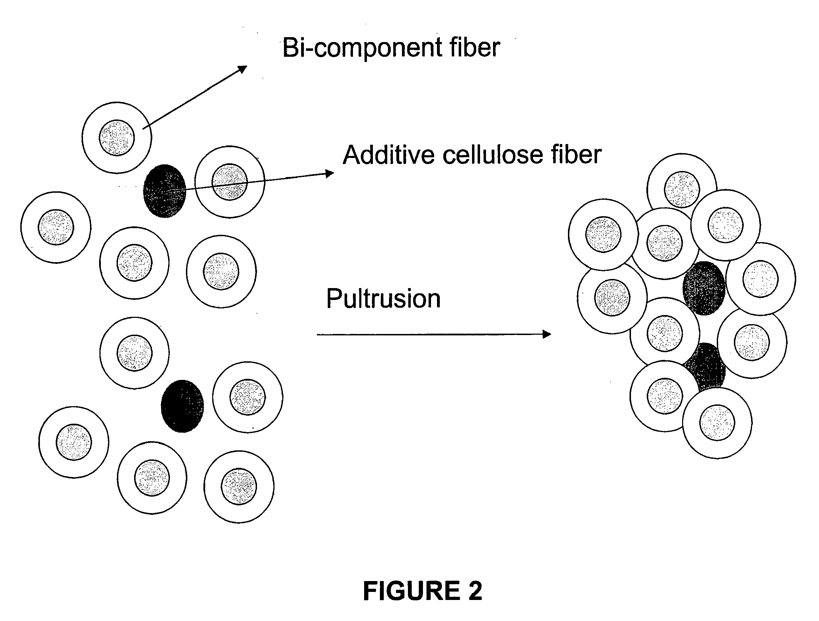

Figure 2 is a schematic representation of bicomponent fibers (sheath and core) and cellulose fibers (dark circles) that are subjected to pultrusion in which sheaths of bicomponent fibers melted and adhered to other sheaths of bicomponent fibers and in some instances to the cellulose fibers.

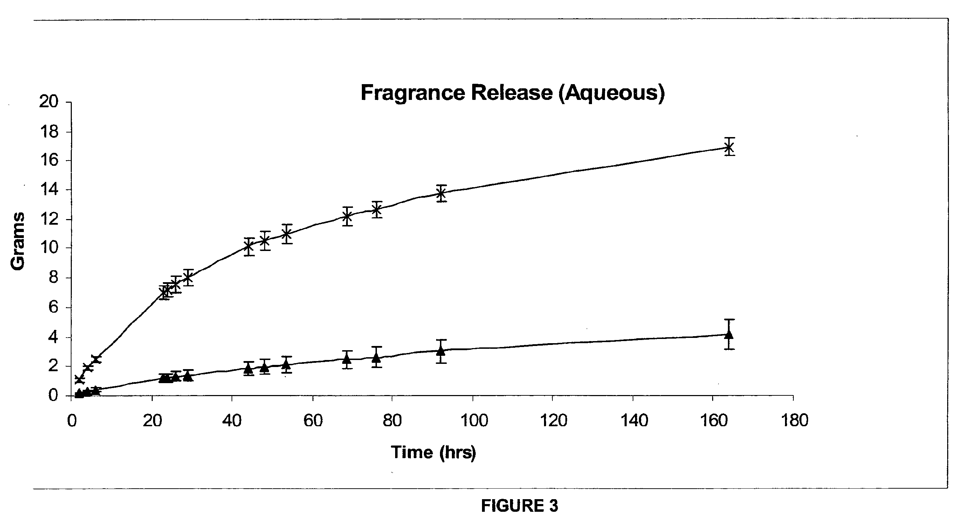

Figure 3 (comparison) is a schematic representation of the total amount fragrance delivered (gm) wicks during a period of time (hours) for 6 wicks (x) and 6 rattan wicks (solid triangle) (both types of wicks are 152.4 mm (6 inches) long) for an aqueous based fragrance. The wicks (x) released significantly more aqueous fragrance that the reed wicks. The wicks (x) in this experiment were made from 90% polyethylene (PE)/polyethylene terephthalate (PET) (PE/PET) fiber and 10% naturally colored cotton fiber. The wicks were activated with oxygen plasma. The wick diameter was 2.79 mm (0.110 inches).

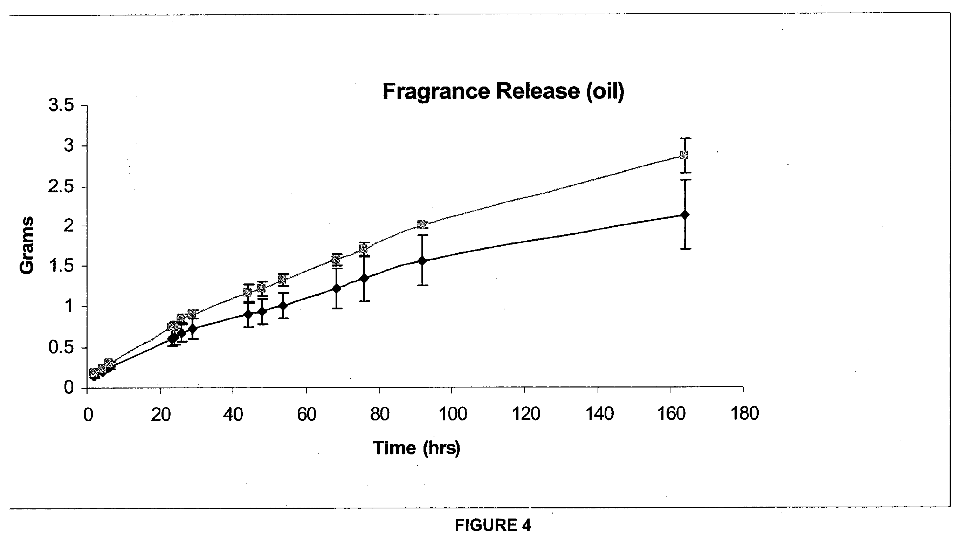

Figure 4 (comparison) is a schematic representation of the total amount fragrance delivered (gm) wicks during a period of time (hours) for 6 wicks (squares) and 6 rattan wicks (solid diamond) (both types of wicks are 6 inches long) for an oil based fragrance. The wicks (squares) released significantly more oil based fragrance that the reed wicks. The wicks (square) in this experiment were made from 90% polyethylene (PE)/polyethylene terephthalate (PET) (PE/PET) fiber and 10% naturally colored cotton fiber. The wicks were activated with oxygen plasma. The wick diameter was 2.79 mm (0.110 inches).

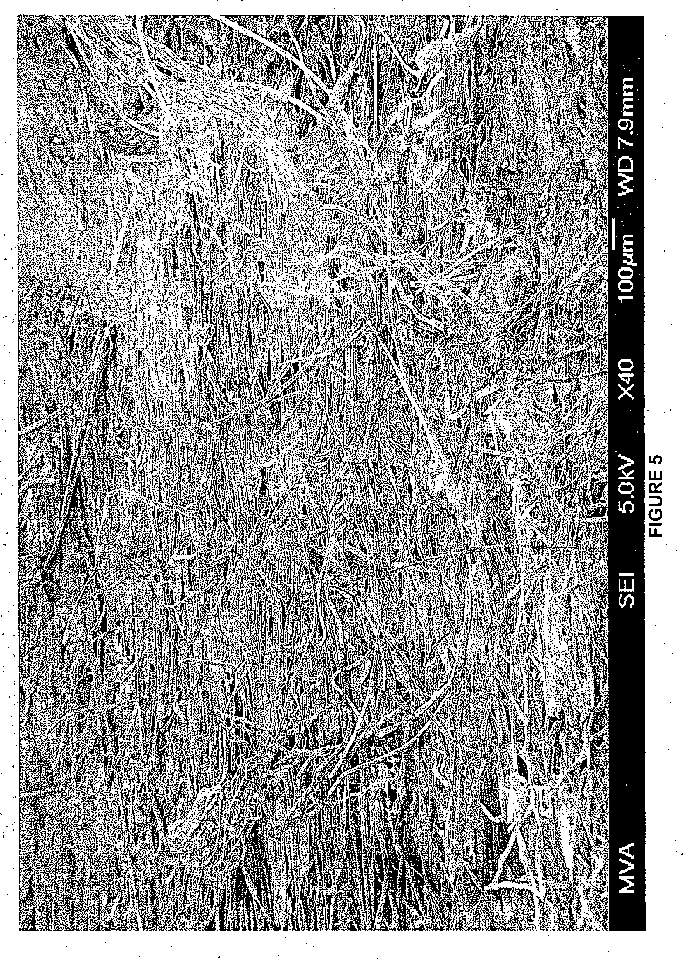

Figure 5 (comparison) is a scanning electron micrograph of the surface of a wick demonstrating the void-spaces (pores) between the fibers. The wicks in this experiment were made from 90% polyethylene (PE)/polyethylene terephthalate (PET) (PE/PET) fiber and 10% naturally colored cotton fiber. The wicks were activated with oxygen plasma. The wick diameter was 2.79 mm (0.110 inches).

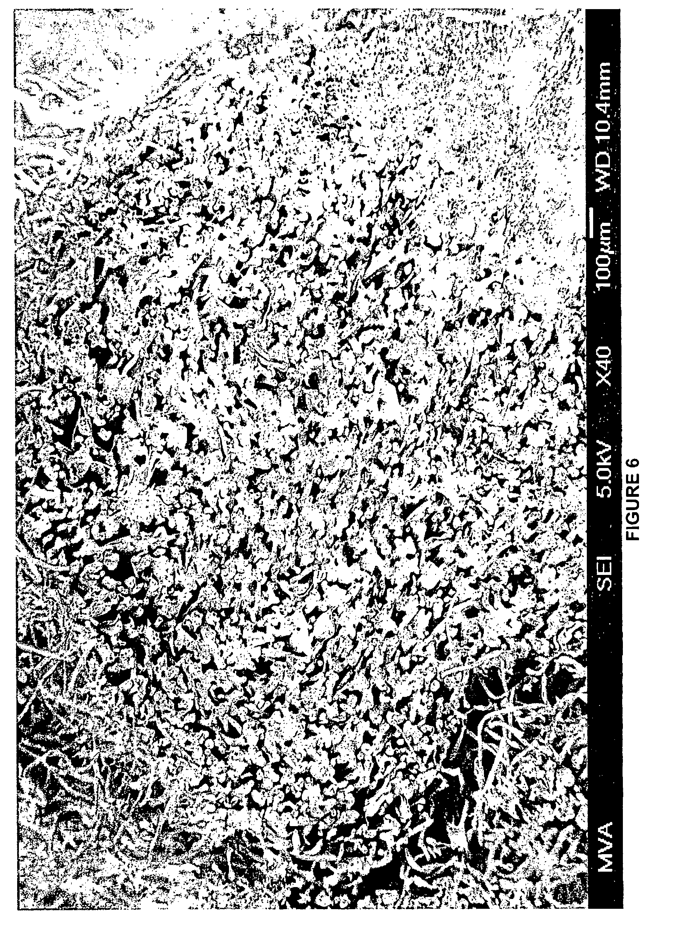

Figure 6 is a scanning electron micrograph of a cross section through the wick shown in Figure 5 demonstrating the void spaces (pores) between the fibers.

### DETAILED DESCRIPTION OF THE INVENTION

This invention provides a hydrophilic porous fiber wick. This invention also provides a biodegradable porous fiber wick. This invention also provides a hydrophilic, biodegradable porous fiber wick. This invention provides biodegradable porous media. The wicks of the present invention may be made from different materials and may be used for delivering vaporizable material, such as fragrance, into the atmosphere, such as the atmosphere of a room environment. Accordingly, the use of the term "fragrance wick" in this application is not limiting to wicks for releasing fragrance but includes wicks that can release other vaporizable materials. Aqueous based fragrances in this invention are fragrance formulations that have a water composition over 50% by weight. The wicks of the present invention may also deliver non-aqueous based fragrances into an environment. These wicks may be made of synthetic fiber materials, or a blend of synthetic fiber materials and natural fiber materials. In one embodiment, synthetic fiber materials comprise synthetic sinterable bicomponent fibers. Synthetic fibers may be biodegradable or non-biodegradable. In another embodiment, synthetic bicomponent fibers are combined with natural monocomponent fibers or synthetic monocomponent fibers to form a wick. In one embodiment, natural fiber materials comprise cotton. In one embodiment, either the synthetic fiber materials, the natural fiber materials or both may be colored. The wicks of the present invention may be colored.

The term biodegradable is used in this application to indicate that a component of the wick is biodegradable. In one embodiment, the wt% of the component of the wick that is biodegradable is at least 40%, at least 50%, at least 60%, at least 70%, at least 80% or at least 90% of the total weight of the wick. In one embodiment, the majority component of the wick is biodegradable.

Sinterable synthetic bicomponent fibers can be partially melted by heating and fused together with void spaces (pores) among the fibers. One component in the sinterable bicomponent fiber has a lower melting temperature than another component in the sinterable bicomponent fiber. Bicomponent fibers can be fused together by melting the lower melting temperature component thereby forming porous media.

In one embodiment, wicks are made of sinterable synthetic bicomponent fibers. In another embodiment, the wicks are made from a two component fiber blend of a sinterable synthetic bicomponent fiber and a monocomponent fiber that is carded into a sliver which is subsequently subjected to heat and pressure in an oven pultrusion process. Monocomponent fibers may be natural or synthetic. In some embodiments, bicomponent fibers may be blended with monocomponent fibers in a weight to weight ratio of about 9.5:1 9:1, 8 :1, 7:1, 6:1, 5:1, or 4:1, or any intermediate number between these ratios (bicomponent fibers:monocomponent fibers). A die on the output side of the oven forms rods of desired diameter that are subsequently cut into wicks. The sheath or core of the bicomponent fiber and/or the monocomponent fiber may be colored. The majority of the fiber blend is composed of the bicomponent synthetic fiber (about 51 wt% to about 95 wt%). The bicomponent synthetic fiber may be at least more than 50 wt%, 60 wt%, 70 wt%, 80 wt%, 90 wt% or 95 wt% of the weight of the wick. The minor component of the fiber blend is a monocomponent fiber which may be colored. This monocomponent fiber is a synthetic fiber or a natural fiber such as a cotton fiber. The monocomponent fiber can be a solution dyed synthetic, reactive dyed synthetic, reactive dyed cotton, genetically modified colored cotton or naturally colored cotton. In another embodiment, wicks are made using solution dyed bicomponent fiber. The color may also be produced by application of a dye solution after the wick is made.

In one embodiment, for carding purposes, the bicomponent fibers and monocomponent fibers should be of similar denier (2-12 denier), length (about 12.7 mm (0.5 inches) to about 76.2 mm (3.0 inches)), and crimp. The monocomponent fibers have a melt or decomposition temperature at least about 10°C higher than that of the bicomponent sheath material. In other embodiments, the monocomponent fibers have a melt or decomposition temperature at least about 20°C higher or at least about 30°C higher than that of the bicomponent sheath material.

In one embodiment, the sliver is bonded together by using an oven pultrusion process. The oven thermally bonds (melts) the sheath material of the bicomponent fibers to other bicomponent fibers and to the non binding monocomponent fibers that do not melt during the pultrusion process. This process produces a cylindrical sintered porous matrix. A die compresses and shapes this matrix into rods that are subsequently air cooled and cut to length.

To improve the wicking of water based fragrances, and other vaporizable materials, procedures have been developed to increase the surface energy of the fiber wick material. One way to increase this surface energy is to treat wicks using plasma. Plasma treatment of the wicks of the present invention is optional. This can be a batch process at low pressure or an inline process at or above atmospheric pressure. A number of gases can be energized to react with the surface of the fiber to create hydrophilic moieties. Gases include, but are not limited to, oxygen, air, nitrogen, and argon. Other inert gases or vapors such as helium, water, or methanol can be used. Various exposure times, pressures, and energies are used during the plasma process depending on the desired product requirements. Experimental results have shown that plasma treating sintered fiber wicks increases the capillary rise height and rate of water based fragrance fluids within the fiber wick. In several embodiments, deionized water moved against gravity at the rate of over about 10.16 mm/minute (0.4 inches/minute) in the initial 50.8 mm (2 inches) of the wick. Plasma treatment increased the water wicking rate of some wicks of the present invention from about 38.1 mm (1.5 inches) in 2 minutes to about 63.5 mm (2.5 inches) in 2 minutes. The PLA/PLA bicomponent fiber is a type of polyester and polyester materials are known to respond well to oxygen plasma treatment processes both in terms of hydrophilic wicking improvement and shelf life.

In another embodiment, finishing agents may be employed to render the wicks more hydrophilic. In one embodiment, bicomponent fibers and/or monocomponent fibers are treated with finishing agents before carding and pultrusion. Bicomponent fibers and/or monocomponent fibers treated with finishing agents are commercially available.

### Synthetic fiber materials

Synthetic fiber materials that can be used to make the wicks of the present invention may be biodegradable or non-biodegradable.

Synthetic biodegradable fibers include but are not limited to the following: poly (lactic acid) (PLA), polyhydroxyalkanoates (PHA), polyhydroxybutyrate-valerate (PHBV), and polycaprolactone (PCL).

### Bicomponent Fibers

Sinterable synthetic bicomponent fibers, according to some embodiments of the present invention, have a core/sheath or side by side cross-sectional structure. In other embodiments, bicomponent fibers have an islands-in-the-sea, matrix fibril, citrus fibril, or segmented pie cross-sectional structure. Bicomponent fibers comprising core/sheath cross-sectional structure are provided in Table 1.

**Table 1 Bicomponent Fibers**

| Sheath | Core |
|---|---|
| polyethylene (PE) | polypropylene (PP) |
| ethylene-vinyl acetate copolymer (EVA) | polypropylene (PP) |
| polyethylene (PE) | polyethylene terephthalate (PET) |
| polyethylene (PE) | polybutylene terephthalate (PBT) |
| polypropylene (PP) | polyethylene terephthalate (PET) |
| polypropylene (PP) | polybutylene terephthalate (PBT) |
| polyethylene (PE) | Nylon-6 |
| polyethylene (PE) | Nylon-6,6 |
| polypropylene (PP) | Nylon-6 |
| polypropylene (PP) | Nylon-6,6 |
| Nylon-6 | Nylon-6,6 |
| Nylon-12 | Nylon-6 |
| copolyester (CoPET) | polyethylene terephthalate (PET) |
| copolyester (CoPET) | Nylon-6 |
| copolyester (CoPET) | Nylon-6,6 |
| glycol-modified PET (PETG) | polyethylene terephthalate (PET) |
| polypropylene (PP) | poly-1,4-cyclohexanedimethyl (PCT) |
| polyethylene terephthalate (PET) | poly-1,4-cyclohexanedimethyl (PCT) |
| polyethylene terephthalate (PET) | polyethylene naphthalate (PEN) |
| Nylon-6,6 | poly-1,4-cyclohexanedimethyl (PCT) |
| polylactic acid (PLA) | polystyrene (PS) |
| polyurethane (PU) | acetal |

In some embodiments, fibers comprise continuous fibers. In other embodiments, fibers comprise staple fibers. In one embodiment, for example, a fiber of a fibrous material comprises a staple bicomponent fiber. Staple fibers, according to some embodiments, have any desired length. In some embodiments, fibrous materials are woven or non-woven. In one embodiment, a fibrous material is sintered. In one embodiment, fibrous wicks are optionally colored.

In one embodiment, synthetic bicomponent fibers are used. Bicomponent fibers that may be used in the present invention may have a variety of configurations such as a concentric sheath and core arrangement, an eccentric core relative to the sheath, a side by side arrangement of fibers, and other configurations known to one of ordinary skill in the art. In other embodiments, bicomponent fibers have an islands-in-the-sea, matrix fibril, citrus fibril, or segmented pie cross-sectional structure. Bicomponent fibers that can be used to make the wicks of the present invention may be sinterable. Synthetic sinterable and generally non-biodegradable bicomponent fibers that may be employed in the practice of this invention include, but are not limited to fibers constructed from the following pairs of polymers: polypropylene/polyethylene terephthalate (PET), polyethylene (PE)/PET, polypropylene/Nylon-6, Nylon-6/PET, copolyester/PET, copolyester/Nylon-6, copolyester/Nylon-6,6, poly-4-methyl-1-pentene/ PET, poly-4-methyl-1-pentene/Nylon-6, poly-4-methyl-1-pentene/Nylon-6,6, PET/polyethylene naphthalate (PEN), Nylon-6,6/poly-1,4-cyclohexanedimethy- 1 (PCT), polypropylene/polybutylene terephthalate (PBT), Nylon-6/co-polyamide, polyester/polyester and polyurethane/acetal.

### Natural fiber materials

Natural fiber materials can be used in combination with the synthetic bicomponent fibers to make the wicks of the present invention. These natural fiber materials include, but are not limited to: cotton fiber, Rayon, Tencel, silk, and wool. Cottons can be any type of cotton, including Pima, Egyptian Cotton, Upland cotton, and Asiatic cotton. Naturally biodegradable cellulose based fibers include vegetable fibers, wood fibers, animal fibers and some man-made cellulose based fibers. Vegetable fibers include cotton fibers.

The cotton can be purchased commercially from many sources such as Cotton Works Inc. (Gaffney, SC, USA), Frontier Spinning Mills Inc. (Stanford, NC, USA), and Parkdale (Gastonia, NC, USA). The are also a number of companies that dye cotton. Cottons can also be dyed with dyes by many methods. One requirement for the dyed cotton for this application is good color fastness. The dye should generally stay on the cotton surface and not move with the aqueous or non-aqueous based fragrances. One type of dye for this application is a reactive dye that can form covalent bonds with cotton. The reactive dyes that can be used in this application include, but are not be limited to Procion MX series dyes, Cibacron F, Drimarene K, Remazol or vinyl sulfone dyes, Levafix, Procion H and H-E, or Novacron from Huntsman chemicals.

The fiber can be long or short. It is important to have similar lengths of the natural fiber and of the synthetic bicomponent fiber. Synthetic fibers can be cut to a desired length. A good match provides better blending (dispersion of the lower concentrate fiber) and better carding. Furthermore, this yields adequate tensile strength for the pultrusion process. Fibers that are too short will not be carded properly into adequate sliver for the pultrusion process. The fibers used in this application are generally from about 12.7 mm to about 63.5 mm (about 0.5 to about 2.5 inches) in length. In one embodiment cotton fibers are from about 12.7 mm to about 38.1 mm (about 0.5 to about 1.5 inches) in length. In one embodiment synthetic bicomponent fibers are from about 12.7 mm to about 63.5 mm (about 0.5 to about 2.5 inches) in length, from about 25.4 mm to about 38.1 mm (about 1.0 to about 1.5 inches) in length, or from about 38.1 mm to about 50.8 mm (about 1.5 to about 2.0 inches) in length.

The non-sintered colored fibrous components that may be employed in the practice of this invention include, but are not limited to: naturally colored cotton fiber (Vreseis Ltd. trade name: Fox fiber), and dye colored cotton, Rayon, Tencel, silk, wool, polyvinyl alcohol (PVA) or acrylic fibers. In a further embodiment, a fibrous component comprises any type or combination of colored fibers known to one of skill in the art useful in aqueous and non-aqueous fragrance wicks.

### Processing Fibers into Wicks

Generally, the synthetic bicomponent fiber materials, and blends of synthetic bicomponent fiber materials with synthetic monocomponent or natural fiber monocomponent materials, used to make the wicks of the present invention, were carded into sliver. The sliver was bonded together by using an oven pultrusion process. In some embodiments, the synthetic biodegradable bicomponent fibers were composed of a concentric sheath and core material. To facilitate sintering, the sheath material was of a lower melting point than the core material. The oven temperature was set between the melting temperature of the sheath and core and the oven thermally bonded (melted) the sheath material of the biodegradable bicomponent fibers to other biodegradable bicomponent fibers. This process produced a cylindrical sintered porous matrix. A die compressed and shaped this matrix into rods that were subsequently air cooled and cut to desired length.

### Optional Plasma Treatment

Optionally, the wicks were plasma treated. Plasma treatment could be any one of commonly employed industrial plasma processes, such as radiofrequency (RF) or microwave plasma. The plasma could also be a low pressure or normal pressure air plasma process. In this specific application, plasma was a low pressure, gas plasma treatment process. The wicks were placed in a chamber for a specified time, energy level, and gas flow rate. The plasma process made the wicks more hydrophilic. The gas was oxygen, but other gases could be used, such as nitrogen, argon, hydrogen and any combination thereof. Other molecules, such as alcohol or acrylic acids also could be used in the plasma chamber to make the polymer more hydrophilic. The gas flow rate was controlled to maintain the chamber at a pressure about 13.3 Pa (100 mtorr) and treatment time generally was a few minutes to 30 minutes. It is widely known that plasma treatment conditions depend on the machine design, sample size, power etc. One of ordinary skill in the art can modify conditions for different component parts and on different plasma machines. A plasma treatment device that feeds inline to the pultrusion process and does not require vacuum conditions and operates at positive pressures (above ambient atmospheric pressure) may be used.

The plasma treatment process creates hydrophilic moieties on the surface of the fiber molecules. These moieties increase the surface energy of the fiber wicks making them more hydrophilic. The cross sectional area determines the amount of fluid that can be transported through the wicks for a given wick density. Larger diameter wicks can transport more fluid.

### Optional Use of Finishing Agents

Finishing agents are optionally employed to enhance hydrophilicity of the wicks. Finishing agents are well known in the textile industry as aiding agents for the fiber process or provide fiber with desired properties, such as water absorption etc. Finishing agents that may be employed were published in the WO/1993/017172, US 4,098,702, and US 4,403,049. Details of the application of finishing agents, including surfactant, to provide textiles with desired properties may be found in "Handbook of Detergents: Formulation" edited by Michael Showell, pages 279-304, CRC Press. Application of finishes may generally be accomplished by contacting a fiber tow or yarn with a solution or emulsion comprising at least one finishing agent having desirable lubrication, antistatic, wetting, and/or emulsification properties. Other additives such as antioxidants, biocides, anti-corrosion agents, and pH control agents may also be added into the finishes. A suitable fiber finish may also be sprayed or applied directly onto fibers or yarn. Fibers treated with finishing agents are commercially available.

### Wicks

The wicks can have different diameters and lengths depending on the desired application. Multiple wicks may be placed in a jar filled with a liquid containing a vaporizable material such as a fragrance. Capillary forces draw the fragrance solution up the wicks. Fragrance is then released by evaporation of the fluid from the surface of the exposed portion of the wicks. One example was a wick with a slender rod 2.03 to 3.81 mm (0.080 to 0.15 inches) in diameter and about 203.2 mm (8 inches) to about 304.8 mm (12 inches) long. In one embodiment, wicks may be made with diameters of about 1.02 mm (0.04 inches) to about 25.4 mm (1 inch) using the pultrusion process. In various embodiments, wick diameters may be about 1.27, 1.52, 1.78, 2.03, 2.29, 2.54, 2.79, 3.05, 3.30, 3.56, 3.81, 4.06, 4.32, 4.57, 4.82, 5.08, 5.33, 5.59, 5.84, 6.10, 6.35, 6.60, 6.86, 7.11, 7.37, or 7.62 mm (0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, 0.25, 0.26, 0.27, 0.28, 0.29, or 0.30 inches). In various embodiments, wick diameters may greater than about 7.62 mm (0.30 inches), greater than about 10.16 mm (0.40 inches), greater than about 12.7 mm (0.50 inches), or greater than about 15.24 mm (0.60 inches). The rods can be cut into wicks of any desired length, for example 5.08 mm (0.2 inches) or greater. In various embodiments, wicks may be equal to or longer than about 50.8 mm (2.0 inches), about 76.2 mm (3.0 inches), about 101.6 mm (4.0 inches), about 127 mm (5.0 inches), about 152.4 mm (6.0 inches), about 177.8 mm (7.0 inches), about 203.2 mm (8.0 inches), about 228.6 mm (9.0 inches), about 254 mm (10 inches), about 279.4 mm (11 inches), about 304.8 mm (12 inches), about 330.2 mm (13 inches), about 355.6 mm (14 inches), about 384 mm (15 inches), about 508 mm (20 inches), about 635 mm (25 inches), or about 762 mm (30 inches). In some embodiments, the wicks have a length to diameter ratio greater than 40, greater than 50, greater than 60, greater than 70, greater than 80, greater than 90, greater than 100, greater than 150, greater than 200, greater than 250, or greater than 300. The wicks of the present invention have a pore volume in the range of about 50% to about 95%.

Release of a vaporizable material, such as a fragrance, through evaporation occurs when the fragrance fluid molecules have sufficient kinetic energy to overcome liquid molecular forces and eject from the wick surface. The exposed surface area of the wicks controls the rate of evaporation. A greater wick surface area (larger diameter and/or length) causes greater fragrance release. Fragrance release is dependent on other environmental factors such as temperature and humidity. The wicks of the present invention function effectively at ambient temperature or room temperature and do not require heat, convection air, or atomization.

The wicks of the present invention can wick vaporizable materials, such as fragrances, for release into the atmosphere. These wicks can wick and release both aqueous and oil based fragrances.

The wicks are not limited to fragrances and may wick and release other vaporizable materials, in addition to or instead of fragrances. Vaporizable materials containing insect repellants may be used to repel undesirable invertebrates, such as mosquitoes, no see ums, flies, wasps, yellow jackets and hornets from the environment. Vaporizable materials containing insecticides may kill insects in the environment. Vaporizable materials containing both a fragrance and an insect repellant or insecticide may be employed for the dual function of a pleasurable odor and insect repellency or an insect kill. Vaporizable fragrances, disinfectants, deodorants, pheromones, insect repellants, and insecticides are well known to one of skill in the art and are available from a variety of commercial sources. Common fragrances comprise citrus oils, fruity floral oils, herbal floral oils, lemon oils, orange oils, or combinations thereof. Disinfectants, in some embodiments, comprise denatonium benzoate, hinokitiol, benzthiazolyl-2-thioalkanoic nitriles, alkyl dimethylbenzyl ammonium chlorides, or trichlosan. Insect repellants, in some embodiments, comprise N,N-diethyl-meta-toluamide, citronella oils, or camphor. Additionally, insecticides, in some embodiments, comprise imiprotrin, cypermethrin, bifentrint, or pyrethrins.

The wick surface energy, density, cross sectional area, and surface area can be optimized for a particular vaporizable material delivery system. The wick can have different colors. The color can be provided by the sinterable bicomponent fiber, by a monocomponent fiber or other additive fibers.

The wick of the present invention may have synthetic and sinterable hydrophilic bicomponent fiber from about 51 wt% to about 100 wt% and other synthetic or natural fibers from about 0 wt% to about 49 wt%. In other embodiments, the wick may have synthetic and sinterable hydrophilic bicomponent fiber from about 60 wt% to about 100 wt%, or 70 wt% to about 100 wt%, and other synthetic or natural fibers from about 0 wt% to about 40 wt% or from about 0 wt% to about 30 wt%, respectfully.

The wick of the present invention may have synthetic biodegradable and sinterable bicomponent fiber from about 51 wt% to about 100 wt% and other synthetic or natural fibers from about 0 wt% to about 49 wt%. In other embodiments, the wick may have synthetic biodegradable and sinterable bicomponent fiber from about 60 wt% to about 100 wt%, or 70 wt% to about 100 wt%, and other synthetic or natural fibers from about 0 wt% to about 40 wt% or from about 0 wt% to about 30 wt%, respectfully.

The wick of the present invention may have synthetic sinterable biodegradable bicomponent fiber from about 51 wt% to about 100 wt% and other natural fibers from about 0 wt% to about 49 wt%. In other embodiments, the wick may have synthetic sinterable biodegradable bicomponent fiber from about 60 wt% to about 100 wt%, or 70 wt% to about 100 wt%, and other natural fibers from about 0 wt% to about 40 wt% or from about 0 wt% to about 30 wt%, respectfully.

The wick of the present invention may have synthetic sinterable biodegradable bicomponent fiber from about 51 wt% to about 100 wt% and other synthetic monocomponent fibers from about 0 wt% to about 49 wt%. In other embodiments, the wick may have sinterable synthetic biodegradable bicomponent fiber from about 60 wt% to about 100 wt%, or 70 wt% to about 100 wt%, and other synthetic monocomponent fibers from about 0 wt% to about 40 wt% or from about 0 wt% to about 30 wt%, respectfully.

The wick of the present invention may have synthetic sinterable non-biodegradable bicomponent fiber from about 51 wt% to about 100 wt% and other natural fibers from about 0 wt% to about 49 wt%. In other embodiments, the wick may have sinterable synthetic non-biodegradable bicomponent fiber from about 60 wt% to about 100 wt%, or 70 wt% to about 100 wt%, and other natural fibers from about 0 wt% to about 40 wt% or from about 0 wt% to about 30 wt%, respectfully.

The wick of the present invention may have synthetic sinterable non-biodegradable bicomponent fiber from about 51 wt% to about 100 wt% and other natural fibers from about 0 wt% to about 49 wt%. In other embodiments, the wick may have sinterable synthetic non-biodegradable bicomponent fiber from about 60 wt% to about 100 wt%, or 70 wt% to about 100 wt%, and other natural fibers from about 0 wt% to about 40 wt% or from about 0 wt% to about 30 wt%, respectfully.

In some embodiments, wicks of the present invention may have sinterable bicomponent fiber from 70 to 95 wt % and hydrophilic monocomponent fiber from 5 to 30 percent (wt %). The wicks have a density from about 0.2 g/ml to about 1.0 g/ml, or from about 0.3 g/ml to about 0.9 g/ml or from about 0.4 g/ml to about 0.8 g/ml.

The following examples will serve to further illustrate the present invention and further wicks disclosed herein without, at the same time, however, constituting any limitation thereof. On the contrary, it is to be clearly understood that resort may be had to various embodiments, modifications and equivalents thereof which, after reading the description herein, may suggest themselves to those skilled in the art, and which are defined by the claims.

### Example 1

### Wicks with synthetic sinterable, biodegradable bicomponent fiber

The wicks were made from pultrusion of synthetic sinterable poly(lactic acid) (PLA) or its copolymer concentric bicomponent fibers. In a specific embodiment, both core and sheath materials were PLA and the core PLA had a melting temperature higher than the melting temperature of sheath PLA (Far Eastern Textile Ltd. Ingeo SLN2450CM, 4 denier). It is preferred that the melting temperature difference is more than 10° C, more than 20°C or more than 30°C. The melting temperature of the polymer can be controlled by manipulation of crystallization, the copolymerization or the blend as known to one of ordinary skill in the art of polymer chemistry.

The sliver was bonded together by using an oven pultrusion process. The synthetic biodegradable bicomponent fibers were composed of a concentric sheath and core material. To facilitate sintering, the PLA in the sheath material was of a lower melting point than the PLA in the core material. For this synthetic biodegradable bicomponent fiber, the melting point for the PLA sheath was about 132 °C and melting point for the PLA in the core was about 165 °C. The oven temperature was controlled based on the manufacturing conditions. The temperature depended on the pultrusion speed and rod diameter. The goal was to provide a sufficient amount of heat to the sinterable bicomponent fiber such that only the sheath of the bicomponent fiber melted but not the core. The silver was pultruded through an oven at the temperature of 204-221°C and compressed through a die at the temperature of 49-66°C. The pultrusion speed was 50.8 to 101.6 mm/seconds (2.0 to 4.0 inches/seconds). This process produced a cylindrical sintered porous matrix. A die compressed and shaped this matrix into rods that were subsequently air cooled and cut to length.

Optionally, the wicks were plasma treated. In this example, plasma was a low pressure, gas plasma treatment process. The wicks were placed in a chamber for a specified time, energy level, and gas flow rate. The plasma process made the wicks more hydrophilic. The gas was oxygen. The gas flow rate was controlled to maintain the chamber at a pressure about 13.3 Pa (100 mtorr) and treatment time generally was a few minutes.

**Table 2**

| Aqueous based fragrance delivery amount (in grams (gm)) for ten 203.2-mm 3.81 mm (8-inch 0.15 inch) diameter wicks produced as described in this example under different pultrusion speeds | | |
|---|---|---|
| | PLA WICKS | |
| Days | Speed One (gm) | Speed Two (gm) |
| 0 | 0 | 0 |
| 3 | 19.51 | 20.92 |
| 5 | 22.99 | 24.39 |
| 6 | 24.18 | 25.55 |
| 7 | 25.19 | 26.54 |
| 10 | 26.91 | 28.16 |
| 21 | 27.63 | 28.84 |
| 22 | 28.08 | 29.28 |
| 23 | 28.64 | 29.80 |
| 24 | 29.01 | 30.14 |
| 27 | 30.42 | 31.47 |
| 28 | 30.99 | 32.04 |
| 29 | 31.56 | 32.57 |
| 30 | 31.95 | 32.95 |
| 31 | 32.44 | 33.39 |
| 34 | 33.67 | 34.51 |
| 35 | 33.99 | 34.79 |

### Example 2

### Wicks with synthetic sinterable biodegradable bicomponent fiber and colored, naturally biodegradable cellulose based fiber.

The wicks were made from pultrusion of a blend of synthetic sinterable poly(lactic acid) (PLA)/PLA concentric bicomponent fibers and a colored naturally biodegradable fiber, such as cotton fiber. These materials were blended in a 9:1 (PLA: cotton) ratio and carded into sliver. The lower content brown cotton provided the natural color of the wicks.

The silver was pultruded through an oven at the temperature of 204-221°C and compressed through a die at the temperature of 49-66°C. The pultrusion speed was 50.8 to 101.6 mm/seconds (2.0 to 4.0 inches/seconds). This process produced a cylindrical sintered porous matrix. A die compressed and shaped this matrix into rods that were subsequently air cooled and cut to length.

Optionally, the wicks were plasma treated. In this specific application, plasma was a low pressure, gas plasma treatment process. The wicks were placed in a chamber for a specified time, energy level, and gas flow rate. The plasma process made the wicks more hydrophilic. The gas was oxygen. The gas flow rate was controlled to maintain the chamber at a pressure about 13.3 Pa (100 mtorr) and treatment time generally was a few minutes.

The wicks were hydrophilic and deionized water wicked to one inch high against gravity in less than 120 seconds.

### Example 3

### Wicks with synthetic sinterable biodegradable bicomponent fiber and hydrophilic synthetic fiber

The wicks are also made from combining synthetic sinterable biodegradable concentric bicomponent fibers with non-sinterable, hydrophilic monocomponent fibers, such as PVA (polyvinyl alcohol) or acrylic fiber materials, that are blended in a 9 to 1 (9: 1) ratio and carded into sliver. The sliver is bonded together by using an oven pultrusion process. The silver is pultruded through an oven at the temperature of 204 to 221 °C and compressed through a die at the temperature of 49 to 66°C. The pultrusion speed was 50.8 to 101.6 mm/seconds (2.0 to 4.0 inches/seconds).

Optionally, the wicks are plasma treated. In this specific application, plasma is a low pressure, gas plasma treatment process. The wicks are placed in a chamber for a specified time, energy level, and gas flow rate. The plasma process makes the wicks more hydrophilic. The gas is oxygen. The gas flow rate is controlled to maintain the chamber at a pressure about 13.3Pa (100 mtorr) and treatment time generally is a few minutes.

### Example 4

### Wicks with sinterable biodegradable bicomponent fiber and dye colored cotton fiber

The sliver contains synthetic sinterable biodegradable bicomponent poly(lactic acid) PLA/PLA fiber (Far Eastern Textile Ltd. Ingeo SLN2450CM, 4 denier) and dye colored cotton fiber (Memphis Upland long staple cotton, dyed by Littlewood Corp. using Novacron dyes manufactured by Huntsman). The cotton is brown in color. The ratio of synthetic sinterable biodegradable bicomponent PLA/PLA fiber to dye cotton fiber is 9:1 by weight.

The silver is pultruded through an oven at the temperature of 204-221°C and compressed through a die at the temperature of 49-66°CC. The pultrusion speed is 50.8 to 101.6 mm/seconds (2.0 to 4.0 inches/seconds). The product (rod) has a light brown marbled appearance similar to balsa wood.

The products are cut into 203.2 mm (8 inch) long wicks and placed in a plasma chamber. The gas is oxygen. The wicks are treated at 400 watts for about 15 minutes. The treated wicks are able to wick water to 25.4 mm (1 inch) high in less than 120 seconds.

### Example 5 - not according to present invention

### Wicks with sinterable bicomponent fiber and naturally colored cotton fiber

The wicks were made from combining sinterable polyethylene/polyester (PE/PET) concentric bicomponent fibers with non-sinterable, natural brown cotton fibers (Vreseis Ltd. Trade name: Fox fiber). These materials were blended in a 9:1 ratio and carded into sliver. The lower content brown cotton provided the natural color of the wicks.

The sliver was bonded together by using an oven pultrusion process. The bicomponent fibers were composed of a concentric sheath and core material. To facilitate sintering, the sheath material was of a lower melting point than the core material. The oven thermally bonded (melted) the sheath material of the bicomponent fibers to other bicomponent fibers and to the non binding fibers. These non binding fibers include monocomponent fibers such as naturally colored cotton or dyed cotton. The non binding fibers generally do not melt and bind to each other. The silver was pultruded through an oven at the temperature of 175-220°C and compressed through a die at the temperature of 49-66°C. The pultrusion speed was 50.8 to 101.6 mm/seconds (2.0 to 4.0 inches/seconds). This process produced a cylindrical sintered porous matrix. A die compressed and shaped this matrix into rods that were subsequently air cooled and cut to length.

Next, the wicks were plasma treated. In this specific application, plasma was a low pressure, gas plasma treatment process. The wicks were placed in a chamber for a specified time, energy level, and gas flow rate. The plasma process made the parts more hydrophilic: The gas was oxygen. The gas flow rate was controlled to maintain the chamber at a pressure about 13.3 Pa (100 mtorr) and treatment time generally was about 15 minutes.

### Comparative Test results

Presented in Table 3 are comparative data between two reed wicks and three sintered fiber wicks over a four hour period. The fiber wicks described in this invention draw water based fluid to a much greater height with a greater wick rate. These wicks in Table 3 were brown in color and were made of co-polyethylene:polyethylene terephthalate (CoPE/PET) bicomponent fiber and naturally colored cotton at the ratio of 9:1. Fragrance delivery amount data are in figures 3 and 4 and are expressed in grams (gm).

**Table 3**

| | | Initial Weight gm | Wick Height (inches) mm | Final Weight gm | Net Weight gm | Wick Rate gm/4hr |
|---|---|---|---|---|---|---|
| Reed | 1 | 0.50 | (2.31) 58.67 | 1.00 | 0.50 | 0.13 |
| Reed | 2 | 0.50 | (2.10) 53.34 | 1.00 | 0.50 | 0.13 |
| Fiber | 1 | 1.50 | (7.98) 202.69 | 2.50 | 1.00 | 0.25 |
| Fiber | 2 | 1.50 | (8.23) 209.04 | 2.50 | 1.00 | 0.25 |
| Fiber | 3 | 1.50 | (8.57) 217.68 | 2.50 | 1.00 | 0.25 |

### Example 6 - not according to present invention

### Wicks with sinterable bicomponent fiber and dye colored cotton fiber

The wicks were made from combining sinterable polyethylene/polyester (PE/PET) bicomponent 3.0 Dtex (diameter)) (Trevira GMBH, Germany) and dye colored cotton fiber (50 mm long length Pima cotton, dyed by Littlewood Corp. using NovaChrome dyes manufactured by Huntsman (Woodlands, Texas, US). The cotton was brown in color. These materials were blended in a 9:1 ratio and carded into sliver. The lower content dyed cotton fibers provided the color of the wicks.

The sliver was bonded together by using an oven pultrusion process. The bicomponent fibers were composed of a concentric sheath and core material. To facilitate sintering, the sheath material was of a lower melting point than the core material. The oven thermally bonded (melted) the sheath material of the bicomponent fibers to other bicomponent fibers and to the non binding fibers. These non binding fibers were dyed cotton fibers. The non binding fibers generally do not melt and bind to each other. The silver was pultruded through an oven at the temperature of 175-220°C and compressed through a die at the temperature of 49-66°C. The pultrusion speed was 50.8 to 101.6 mm/second (2.0 to 4.0 inches/second). This process produced a cylindrical sintered porous matrix. A die compressed and shaped this matrix into rods that were subsequently air cooled and cut to length. The resulted wick had the color of the dyed cotton.

Next, the wicks were plasma treated in the similar way as naturally colored wicks. The gas flow rate was controlled to maintain the chamber at a pressure about 13.3 Pa (100 mtorr) and treatment time was about 15 minutes.

The wick can have different diameters and lengths depending on the application. Multiple wicks were placed in a jar filled with a fragrance liquid. Capillary forces drew the fragrance solution up the wicks. Fragrance was then released by evaporation of the fluid from the surface of the exposed portion of the wicks. Ten wicks were placed in a small jar (approx. 76.2 to 152.4 mm (3 to 6 inches) tall), filled with a fragrance liquid and a portion of the length of wicks was submerged in the solution.

The ratio of the bicomponent fiber and dye colored cotton fiber was 9:1. However this ratio can be changed accordingly based on the application.

**Table 4**

| Aqueous based fragrance delivery amount (in grams (gm) for ten 203.2-mm (8-inch) long, 2.79 mm (0.11 inch) diameter wicks produced as described in this example and ten 203.2-mm (8-inch) long rattan wicks in 35 days. | | |
|---|---|---|
| Day | Invention Wick· gm | Rattan gm |
| 0 | 0 | 0 |
| 1 | 6.12 | 1.19 |
| 2 | 8.97 | 1.82 |
| 6 | 13.85 | 3.36 |
| 7 | 14.50 | 3.64 |
| 8 | 15.08 | 3.92 |
| 9 | 15.62 | 4.18 |
| 12 | 17.01 | 4.91 |
| 14 | 17.68 | 5.29 |
| 15 | 18.02 | 5.49 |
| 16 | 18.39 | 5.70 |
| 19 | 19.02 | 6.13 |
| 20 | 19.31 | 6.36 |
| 21 | 19.51 | 6.52 |
| 22 | 19.78 | 6.71 |
| 23 | 19.96 | 6.85 |
| 24 | 20.75 | 7.39 |
| 27 | 21.13 | 7.65 |
| 28 | 21.53 | 7.89 |
| 29 | 21.79 | 8.05 |
| 30 | 22.12 | 8.25 |
| 31 | 22.99 | 8.80 |
| 34 | 23.23 | 8.94 |
| 35 | 23.23 | 8.94 |

### Example 7 - not according to present invention

### Wicks with sinterable bicomponent fiber and dye colored protein fiber

The wicks are also made from combining sinterable polyethylene/polyester (PE/PET) concentric bicomponent fibers with non-sinterable, dyed protein-based fibers, such as silk or wool. These materials are blended in a 9:1 ratio and carded into sliver. The lower content of dyed fiber provides the color of the wicks.

### Example 8 - not according to present invention

### Wicks with sinterable bicomponent fiber and dye colored cellulosic fiber

The wicks are also made from combining sinterable polyethylene/polyester (PE/PET) concentric bicomponent fibers with non-sinterable, dyed cellulose based fibers, such as Rayon, Tencel, or wood fibers. These materials are blended in a 9:1 ratio and carded into sliver. The lower content of dyed cellulose fiber provides the color of the wicks.

### Example 9 - not according to present invention

### Wicks with sinterable bicomponent fiber and dye colored hydrophilic synthetic fiber

The wicks are also made from combining sinterable polyethylene/polyester (PE/PET) concentric bicomponent fibers with non-sinterable, hydrophilic dyed synthetic fibers, such polyvinyl alcohol (PVA) and acrylic. These materials are blended in a 9:1 ratio and carded into sliver. The lower content of dyed hydrophilic synthetic fiber provides the color of the wicks.

### Example 10 - not according to present invention

### Wicks with sinterable solution dyed bicomponent fiber and hydrophilic fiber

The wicks are also made from combining sinterable colored concentric bicomponent fibers such as polyethylene/polyester (PE/PET), or polyester/polyester fibers, with a hydrophilic cellulosic, protein or synthetic fiber, such as cotton, Rayon, Tencel, silk, wool, PVA or polyacrylate fibers. These materials are blended in a 9:1 ratio (bicomponent fiber: hydrophilic fibers) and carded into sliver. The high content of colored bicomponent fiber provides the color of the wicks. Usually there is an economic advantage in using dyed cotton as opposed to solution dyed synthetic fiber.

Wicks are optionally plasma treated after the pultrusion process to provide the wicks with satisfactory fragrance delivery rate. The plasma treatment may occur minutes to days following the pultrusion process.

### Example 11 - not according to present invention

### Wicks with specially finished hydrophilic sinterable bicomponent fiber

The sliver contained sinterable bicomponent fiber (co polyethylene (PE)/PET bicomponent 3.0 Dtex (diameter)) (Kosa, USA) GMBH, Germany). The silver was pultruded through an oven at the temperature of 175-220 °C and compressed through a die at the temperature of 60°C. The pultrusion speed was 50.8-101.6 mm/second (2-4 inch/second).

The products were cut into 203.2 mm (8 inch) long wicks and placed in a plasma chamber. The gas was oxygen. The wicks were treated at 200 watts for about 15 minutes. Fragrance delivery rate for plasma and non-plasma treated wicks were similar.

**Table 5**

| Aqueous based fragrance delivery amount (in grams (gm) for ten 203.2-mm (8-inch) long, 3.81 mm (0.15 inch) diameter wicks produced as described in this example with and without plasma treatment. | | |
|---|---|---|
| Days | Untreated gm | Treated gm |
| 0 | 0 | 0 |
| 3 | 15.52 | 15.57 |
| 5 | 18.17 | 18.35 |
| 6 | 19.23 | 19.50 |
| 7 | 20.27 | 20.50 |
| 10 | 22.37 | 22.29 |
| 21 | 23.33 | 23.06 |
| 22 | 23.98 | 23.57 |
| 23 | 24.79 | 24.19 |
| 24 | 26.91 | 24.64 |
| 27 | 28.96 | 26.30 |
| 28 | 29.73 | 26.97 |
| 29 | 30.46 | 27.63 |
| 30 | 31.03 | 28.11 |
| 31 | 31.75 | 28.70 |
| 34 | 33.39 | 30.24 |
| 35 | 33.80 | 30.65 |

### Example 12 - not according to present invention

### Wicks with synthetic sinterable bicomponent fiber and colored hydrophilic synthetic fiber

The wicks were made by combining synthetic sinterable concentric bicomponent fibers PE/PET bicomponent 3.0 Dtex (diameter)) (Trevira GMBH, Germany) with non-sinterable, hydrophilic monocomponent green acrylic fiber materials blended in a 7 to 3 (7:3) ratio and carded into sliver. The acrylic fibers were modacrylic fibers that were dyed green (available from VMOD Fibers LLC, Charlotte, NC, US). The sliver was bonded together using an oven pultrusion process. The silver was pultruded through the oven at a temperature of 204-221 °C and compressed through a die at a temperature of 49-66°C. The pultrusion speed was 50.8 to 101.6 mm/seconds (2.0 to 4.0 inches/seconds).

Optionally, the wicks were plasma treated. In this specific application, plasma was a low pressure, gas plasma treatment process. The wicks were placed in a chamber for a specified time, energy level, and gas flow rate. The plasma process made the wicks more hydrophilic. The gas was oxygen. The gas flow rate was controlled to maintain the chamber at a pressure about 13.3 Pa (100 mtorr) and treatment time generally was a few minutes. The resulting wicks were green colored, hydrophilic and able to wick water against gravity to 25.4 mm (1 inch) high in less than 120 seconds.

## Claims

1. A hydrophilic porous wick comprising sintered synthetic bicomponent fibers having pores between the sintered synthetic bicomponent fibers, wherein the synthetic bicomponent fibers comprise PLA/PLA bicomponent fibers.

2. The hydrophilic porous wick of claim 1, further comprising natural monocomponent fibers or synthetic monocomponent fibers.

3. The hydrophilic porous wick of claim 1, wherein the synthetic bicomponent fibers are selected from poly (lactic acid) (PLA)/PLA.

4. The hydrophilic porous wick of claim 2, wherein the synthetic monocomponent fibers are selected from the group consisting of acrylic fibers, polyvinyl alcohol (PVA) fibers, poly (lactic acid) (PLA) fibers, polyhydroxyalkanoate (PHA) fibers, polyhydroxybutyrate-valerate (PHBV) fibers and polycaprolactone (PCL) fibers.

5. The hydrophilic porous wick of claim 2, wherein the natural monocomponent fibers are selected from the group consisting of cotton, Rayon, Tencel, silk, and wool or wherein the natural monocomponent fibers are cellulose based fibers selected from the group consisting of vegetable fibers, wood fibers, animal fibers and man-made fibers or wherein the natural monocomponent fibers are selected from the group consisting of reactive dyed cotton, genetically modified colored cotton or naturally colored cotton.

6. The hydrophilic porous wick of claim 2, wherein the sintered synthetic bicomponent fibers comprise over 50 wt % to about 95 wt % of the hydrophilic porous wick.

7. The hydrophilic porous wick of claim 1, wherein the synthetic bicomponent fibers comprise a sheath and a core, and the core has a melting temperature at least more than 10°C higher than the melting temperature of the sheath.

8. The hydrophilic porous wick of any of the preceding claims, wherein (a) the synthetic bicomponent fibers are colored or/and (b) wherein the natural monocomponent fibers or synthetic monocomponent fibers are colored.

9. The hydrophilic porous wick of any of the preceding claims, wherein the wick is biodegradable.

10. The hydrophilic wick of claim 9, wherein a component of the wick that is biodegradable component is at least 40% of the total weight of the wick.

11. The hydrophilic porous wick of any of the preceding claims, having
(a) a density from 0.2 g/ml to 1.0 g/ml or/and
(b) a diameter of about 1.02 mm (0.04 inches) to about 25.4 mm (1.0 inches) and a length to diameter ratio greater than 50 or/and
(c) a wicking rate of deionized water of about 12.7 mm (0.5 inches) per minute or more in the first 50.8 mm (2 inches) of length.

12. The hydrophilic porous wick of claim 9, comprising synthetic biodegradable fibers.

13. The hydrophilic porous wick of claim 12, comprising synthetic biodegradable fibers selected from the group consisting of poly (lactic acid) (PLA) fibers, polyhydroxyalkanoate (PHA) fibers, polyhydroxybutyrate-valerate (PHBV) fibers and polycaprolactone (PCL) fibers.

14. The hydrophilic porous wick of any of the preceding claims, wherein the natural monocomponent fibers are dyed.

15. The hydrophilic porous wick of claim 14, wherein the natural monocomponent fibers are dyed with a reactive dye.

16. The hydrophilic porous wick of any of the preceding claims, wherein the hydrophilic porous wick is plasma treated.

17. A method of releasing a vaporizable material into an atmosphere comprising:
placing the hydrophilic porous wick of any of the preceding claims into a fluid containing a vaporizable material;
permitting the fluid containing the vaporizable material to wick through the hydrophilic porous wick and release into the atmosphere.

18. The method of claim 17, wherein (a) the wick releases more than 0.5 gm of vaporizable material in 24 hours, or (b) the vaporizable material is an aqueous based fragrance or oil based fragrance.

19. The method of claim 17, wherein the fluid is an aqueous based fluid.

## Patentansprüche

1. Hydrophiler poröser Docht, umfassend gesinterte synthetische Zweikomponentenfasern mit Poren zwischen den gesinterten synthetischen Zweikomponentenfasern, worin die synthetischen Zweikomponentenfasern PLA/PLA-Zweikomponentenfasern umfassen.

2. Hydrophiler poröser Docht nach Anspruch 1, weiterhin umfassend natürliche Einkomponentenfasern oder synthetische Einkomponentenfasern.

3. Hydrophiler poröser Docht nach Anspruch 1, worin die synthetischen Zweikomponentenfasern ausgewählt sind aus Poly(milchsäure) (PLA)/PLA.

4. Hydrophiler poröser Docht nach Anspruch 2, worin die synthetischen Einkomponentenfasern ausgewählt sind aus der Gruppe, bestehend aus Acrylfasern, Polyvinylalkohol (PVA)-Fasern, Poly(milchsäure) (PLA)-Fasern, Polyhydroxyalkanoat (PHA)-Fasern, Polyhydroxybutyrat-valerat (PHBV)-Fasern und Polycaprolacton (PCL)-Fasern.

5. Hydrophiler poröser Docht nach Anspruch 2, worin die natürlichen Einkomponentenfasern ausgewählt sind aus der Gruppe, bestehend aus Baumwolle, Rayon, Tencel, Seide und Wolle, oder worin die natürlichen Einkomponentenfasern Fasern auf Cellulosebasis sind, ausgewählt aus der Gruppe, bestehend aus Pflanzenfasern, Holzfasern, tierischen Fasern und Kunstfasern, oder worin die natürlichen Einkomponentenfasern ausgewählt sind aus der Gruppe, bestehend aus Baumwolle mit Reaktivfarbstoff, genetisch modifizierter gefärbter Baumwolle oder natürlich gefärbter Baumwolle.

6. Hydrophiler poröser Docht nach Anspruch 2, worin die gesinterten synthetischen Zweikomponentenfasern über 50 Gew.-% bis etwa 95 Gew.-% des hydrophilen porösen Dochts umfassen.

7. Hydrophiler poröser Docht nach Anspruch 1, worin die synthetischen Zweikomponentenfasern einen Mantel und einen Kern umfassen und worin der Kern eine Schmelztemperatur aufweist, die mindestens mehr als 10 °C höher ist als die Schmelztemperator des Mantels.

8. Hydrophiler poröser Docht nach einem der vorhergehenden Ansprüche, worin (a) die synthetischen Zweikomponentenfasern gefärbt sind oder/und (b) worin die natürlichen Einkomponentenfasern oder synthetischen Einkomponentenfasern gefärbt sind.

9. Hydrophiler poröser Docht nach einem der vorhergehenden Ansprüche, worin der Docht bioabbaubar ist.

10. Hydrophiler poröser Docht nach Anspruch 9, worin eine Komponente des Dochts, die eine bioabbaubare Komponente ist, mindestens 40% des Gesamtgewichts des Dochts umfasst.

11. Hydrophiler poröser Docht nach einem der vorhergehenden Ansprüche mit
(a) einer Dichte von 0,2 g/ml bis 1,0 g/ml oder/und
(b) einem Durchmesser von etwa 1,02 mm (0,04 Zoll) bis etwa 25,4 mm (1 Zoll) und einem Verhältnis von Länge zu Durchmesser von größer als 50 oder/und
(c) einer Dochtwirkung für entionisiertes Wasser von etwa 12,7 mm (0,5 Zoll) pro Minute oder mehr in den ersten 50,8 mm (2 Zoll) Länge.

12. Hydrophiler poröser Docht nach Anspruch 9, umfassend synthetische bioabbaubare Fasern.

13. Hydrophiler poröser Docht nach Anspruch 12, umfassend synthetische bioabbaubare Fasern, ausgewählt aus der Gruppe, bestehend aus Poly(milchsäure) (PLA)-Fasern, Polyhydroxyalkanoat (PHA)-Fasern, Polyhydroxybutyrat-valerat (PHBV)-Fasern und Polycaprolacton (PCL)-Fasern.

14. Hydrophiler poröser Docht nach einem der vorhergehenden Ansprüche, worin die natürlichen Einkomponentenfasern gefärbt sind.

15. Hydrophiler poröser Docht nach Anspruch 14, worin die natürlichen Einkomponentenfasern mit einem Reaktivfarbstoff gefärbt sind.

16. Hydrophiler poröser Docht nach einem der vorhergehenden Ansprüche, worin der hydrophile poröse Docht Plasma-behandelt ist.

17. Verfahren zum Freisetzen eines verdampfbaren Materials in eine Atmosphäre, umfassend:
Anordnen des hydrophilen porösen Dochts nach einem der vorhergehenden Ansprüche in einem Fluid, das ein verdampfbares Material enthält;
Zulassen, dass das Fluid, das das verdampfbare Material enthält, durch den hydrophilen porösen Docht transportiert und in die Atmosphäre freigesetzt wird.

18. Verfahren nach Anspruch 17, worin (a) der Docht mehr als 0,5 g des verdampfbaren Materials in 24 h freigibt, oder (b) das verdampfbare Material ein Duftstoff auf wässriger Basis oder ein Duftstoff auf öliger Basis ist.

19. Verfahren nach Anspruch 17, worin das Fluid ein Fluid auf wässriger Basis ist.

## Revendications

1. Une mèche poreuse hydrophile comprenant des fibres bicomposantes synthétiques frittées présentant des pores entre les fibres bicomposantes synthétiques frittées, dans laquelle les fibres bicomposantes synthétiques comprennent des fibres bicomposantes PLA/PLA.

2. La mèche poreuse hydrophile de la revendication 1, comprenant en outre des fibres monocomposantes naturelles ou des fibres monocomposantes synthétiques.

3. La mèche poreuse hydrophile de la revendication 1, dans laquelle les fibres bicomposantes synthétiques sont choisies parmi les fibres d'acide (poly)lactique (PLA)/PLA.

4. La mèche poreuse hydrophile de la revendication 2, dans laquelle les fibres monocomposantes synthétiques sont choisies parmi le groupe se composant des fibres acryliques, des fibres d'alcool polyvinylique (PVA), des fibres d'acide poly(lactique) (PLA), des fibres de polyhydroxy-alcanoate (PHA), des fibres de polyhydroxybutyrate-valérate (PHBV) et des fibres de polycaprolactone (PCL).

5. La mèche poreuse hydrophile de la revendication 2, dans laquelle les fibres monocomposantes naturelles sont choisies parmi le groupe se composant du coton, de la Rayonne, du Tencel, de la soie et de la laine ou dans laquelle les fibres monocomposantes naturelles sont des fibres à base de cellulose choisies parmi le groupe se composant des fibres végétales, des fibres ligneuses, des fibres animales et des fibres synthétiques ou artificielles ou dans laquelle les fibres monocomposantes naturelles sont choisies parmi le groupe se composant du coton teint par une teinture réactive, du coton génétiquement modifié coloré ou du coton naturellement coloré.

6. La mèche poreuse hydrophile de la revendication 2, dans laquelle les fibres bicomposantes synthétiques frittées comprennent entre plus de 50 % en poids à environ 95 % en poids de la mèche poreuse hydrophile.

7. La mèche poreuse hydrophile de la revendication 1, dans laquelle les fibres bicomposantes synthétiques comprennent une gaine et un noyau, et le noyau présente une température de fusion qui est au moins plus de 10 °C supérieure à la température de fusion de la gaine.

8. La mèche poreuse hydrophile de selon l'une quelconque des revendications précédentes, dans laquelle a) les fibres bicomposantes synthétiques sont colorées ou/et b) dans laquelle les fibres monocomposantes naturelles ou les fibres monocomposantes synthétiques sont colorées.

9. La mèche poreuse hydrophile selon l'une quelconque des revendications précédentes, dans laquelle la gaine est biodégradable.

10. La mèche poreuse hydrophile de la revendication 9, dans laquelle un composant de la mèche qui est un composant biodégradable représente au moins 40 % du poids total de la mèche.

11. La mèche poreuse hydrophile selon l'une quelconque des revendications précédentes, présentant
a) une densité de 0,2 g/ml à 1,0 g/ml ou/et
b) un diamètre compris entre environ 1,02 mm (0,04 pouce) à environ 25,4 mm (1,0 pouce) et un rapport entre la longueur et le diamètre supérieur à 50 ou/et
c) une vitesse d'imprégnation par capillarité d'eau désionisée d'environ 12,7 mm (0,5 pouce) par minute ou plus élevée dans les premiers 50,8 mm (2 pouces) de longueur.

12. La mèche poreuse hydrophile de la revendication 9, comprenant des fibres synthétiques biodégradables.

13. La mèche poreuse hydrophile de la revendication 12, comprenant des fibres synthétiques biodégradables choisies parmi le groupe constitué composant des fibres d'acide poly(lactique) (PLA), des fibres de polyhydroxyalcanoate (PHA), des fibres de polyhydroxybutyrate-valérate (PHBV) et des fibres de polycaprolactone (PCL).

14. La mèche poreuse hydrophile selon l'une quelconque des revendications précédentes, dans laquelle les fibres monocomposantes naturelles sont teintes.

15. La mèche poreuse hydrophile de la revendication 14, dans laquelle les fibres monocomposantes naturelles sont teintes avec une teinture réactive.

16. La mèche poreuse hydrophile selon l'une quelconque des revendications précédentes, dans laquelle la mèche poreuse hydrophile est traitée par plasma.

17. Une méthode de libération d'une substance vaporisable dans une atmosphère comprenant :
placer la mèche poreuse hydrophile selon l'une quelconque des revendications précédentes dans un fluide contenant une substance vaporisable;
laisser le fluide contenant la substance vaporisable imprégner par capillarité au travers de la mèche poreuse hydrophile et se libérer dans l'atmosphère.

18. La méthode de la revendication 17, dans laquelle a) la mèche libère plus de 0,5 g de substance vaporisable en 24 heures, ou b) la substance vaporisable est un parfum à base aqueuse ou un parfum à base huileuse.

19. La méthode de la revendication 17, dans laquelle le fluide est un fluide à base aqueuse.
